# EUROPÄISCHE PATENTSCHRIFT

(11) **EP 3 798 656 B1**
(45) Veröffentlichungstag und Bekanntmachung des Hinweises auf die Patenterteilung: **25.05.2022**
(21) Anmeldenummer: 19200322.6
(22) Anmeldetag: 30.09.2019
(51) Int. Cl.: G01R 33/48

(54) **BESCHLEUNIGTE ERFASSUNG VON MESSDATEN MITTELS MAGNETRESONANZ**
ACCELERATED DETECTION OF MEASUREMENT DATA BY MEANS OF MAGNETIC RESONANCE
DÉTECTION ACCÉLÉRÉE DES DONNÉES MESURÉES PAR RÉSONANCE MAGNÉTIQUE

(43) Veröffentlichungstag der Anmeldung: 31.03.2021
(73) Patentinhaber: Siemens Healthcare GmbH, 91052 Erlangen (DE)
(72) Erfinder: Paul, Dominik, 91088 Bubenreuth (DE); Kartäusch, Ralf, 91088 Bubenreuth (DE); Zeller, Mario, 91054 Erlangen (DE)

(56) Entgegenhaltungen:
- DE-A1-102011 083 619
- US-A1- 2014 084 919
- WEIGER MARKUS ET AL: "Short-T2 MRI: Principles and recent advances", PROGRESS IN NUCLEAR MAGNETIC RESONANCE SPECTROSCOPY, PERGAMON PRESS, OXFORD, GB, Bd. 114, 30. Juli 2019 (2019-07-30), Seiten 237-270, XP085921084, ISSN: 0079-6565, DOI: 10.1016/J.PNMRS.2019.07.001 [gefunden am 2019-07-30]

## Beschreibung

Die Erfindung betrifft eine beschleunigte Erfassung von Messdaten mittels Magnetresonanz, die insbesondere besonders kurze Echozeiten TE (z.B. TE < 500 µs) ermöglicht, sodass auch Messdaten von Stoffen mit einer transversalen Relaxationszeit T2 in einem Bereich von 30 - 80 µs, wie z.B. Knochen, erfasst werden können.

Die Magnetresonanz-Technik (im Folgenden steht die Abkürzung MR für Magnetresonanz) ist eine bekannte Technik, mit der Bilder vom Inneren eines Untersuchungsobjektes erzeugt werden können. Vereinfacht ausgedrückt wird hierzu das Untersuchungsobjekt in einem Magnetresonanzgerät in einem vergleichsweise starken statischen, homogenen Grundmagnetfeld, auch B₀-Feld genannt, mit Feldstärken von 0,2 Tesla bis 7 Tesla und mehr positioniert, so dass sich dessen Kernspins entlang des Grundmagnetfeldes orientieren. Zum Auslösen von als Signale messbaren Kernspinresonanzen werden hochfrequente Anregungspulse (RF-Pulse) in das Untersuchungsobjekt eingestrahlt, die ausgelösten Kernspinresonanzen als sogenannte k-Raumdaten gemessen und auf deren Basis MR-Bilder rekonstruiert oder Spektroskopiedaten ermittelt. Zur Ortskodierung der Messdaten werden dem Grundmagnetfeld schnell geschaltete magnetische Gradientenfelder, kurz Gradienten genannt, überlagert. Die aufgezeichneten Messdaten werden digitalisiert und als komplexe Zahlenwerte in einer k-Raum-Matrix abgelegt. Aus der mit Werten belegten k-Raum-Matrix ist z.B. mittels einer mehrdimensionalen Fourier-Transformation ein zugehöriges MR-Bild rekonstruierbar.

Eine beliebte Pulssequenz zur Anregung und Aufzeichnung der Kernspinresonanzen ist die sogenannte Gradientenecho-Sequenz (GRE-Sequenz), insbesondere zur Aufnahme von dreidimensionalen (3D) Datensätzen. Allerdings sind derartige GRE-basierten MR-Untersuchungen zumeist sehr laut und daher unangenehm für einen zu untersuchenden Patienten. Hauptgrund für die hohe Geräuschentwicklung sind die sich bei GRE-Sequenzen zeitlich schnell ändernden Gradientenkonstellationen und die damit verbundenen hohen Slew-Rates (zeitliche Änderung der Gradientenamplituden dG/dt). Außerdem werden im Protokoll der Sequenz oft Parameter benötigt, die besonders schnelles Schalten der Gradienten erfordern, wie zum Beispiel kurze Echozeiten oder Gradientenspoiling.

Durch ein Erfassen von MR-Daten mit sehr kurzen Echozeiten TE (z.B. TE < 500 µs) bieten sich in der Magnetresonanztomographie neue Anwendungsgebiete. Dadurch ist es möglich, Stoffe oder Gewebe darzustellen, welche mittels herkömmlicher Sequenzen, wie z.B. einer (T)SE-Sequenz ("(Turbo) Spin Echo") oder einer GRE-Sequenz, nicht dargestellt werden können, da ihre T2-Zeit, die Relaxation der Quermagnetisierung dieses Stoffs oder Gewebes, deutlich kürzer als die dort erreichbare kürzeste Echozeit TE ist und somit ein entsprechendes Signal von diesen Stoffen oder Geweben zum Aufnahmezeitpunkt bereits zerfallen ist. Mit Echozeiten, welche im Bereich der entsprechenden Zerfallszeit liegen, wäre es beispielsweise möglich, Knochen, Zähne oder Eis in einem MR-Bild darzustellen, obwohl die T2-Zeit dieser Objekte in einem Bereich von 30 - 80 µs liegt.

Nach dem Stand der Technik sind bereits Sequenzen bekannt, welche eine sehr kurze Echozeit ermöglichen. Ein Beispiel ist die radiale UTE-Sequenz ("Ultrashort Echo Time"), wie sie z.B. in dem Artikel von Sonia Nielles-Vallespin "3D radial projection technique with ultrashort echo times for sodium MRI: Clinical applications in human brain and skeletal muscle", Magn. Res. Med. 2007; 57; S. 74-81, beschrieben wird. Bei diesem Sequenz-Typ werden nach einer Wartezeit T_delay nach einer nicht- oder schichtselektiven Anregung die Gradienten hochgefahren und zeitgleich mit der Datenakquisition begonnen. Die derart abgetastete k-Raum-Trajektorie nach einer Anregung verläuft radial vom k-Raumzentrum nach außen. Daher müssen vor der Rekonstruktion der Bilddaten aus den im k-Raum aufgenommenen Rohdaten mittels Fourier-Transformation diese Rohdaten, z.B. durch Regridding, zunächst auf ein kartesisches k-Raum-Gitter umgerechnet werden.

Ein weiterer Ansatz, um kurze Echozeiten zu ermöglichen, ist es den k-Raum punktartig abzutasten, indem der freie Induktionszerfall (FID ("Free Inducation Decay")) erfasst wird. Ein solches Verfahren wird auch als Einzelpunkt-Bildgebung bezeichnet, da pro RF-Anregung im Wesentlichen nur ein Rohdatenpunkt im K-Raum erfasst wird. Ein Beispiel für ein solches Verfahren zur Einzelpunkt-Bildgebung ist das RASP-Verfahren ("Rapid Single Point (RASP) Imaging", O. Heid, M. Deimling, SMR, 3rd Annual Meeting, Seite 684, 1995). Gemäß dem RASP-Verfahren wird zu einem festen Zeitpunkt nach der RF-Anregung durch einen RF-Anregugnspuls zur "Echozeit" TE ein Rohdatenpunkt im k-Raum ausgelesen, dessen Phase von Gradienten kodiert wurde. Die Gradienten werden mittels der Magnetresonanzanlage für jeden Rohdatenpunkt bzw. Messpunkt geändert und somit der k-Raum Punkt für Punkt abgetastet.

Grodzki et al. beschreiben in "Ultrashort echo time imaging using pointwise encoding time reduction with radial acquisition (PETRA)", Magn. Reson Med. 2012 Feb; 67(2):510-8 ein Verfahren, dass das genannte UTE-Verfahren und ein Einzelpunkt-Bildgebungsverfahren, wie z.B. ein RASP-Verfahren, kombiniert. Die PETRA-Sequenz hat den weiteren Vorteil, dass sie im Gegensatz zu anderen Pulssequenzen (insbesondere im Vergleich zu GRE-Sequenzen) zu einer besonders geringen Geräuschbelastung führt, was für untersuchte Patienten sehr angenehm ist. Dabei ist es allerdings durch die mit dem Akquisitionschema einhergehende Dauer der Datenakquisition nicht möglich, dynamische Vorgänge, wie z.B. eine Kontrastmittelinjektion, abzubilden. Bei der typischen Messzeit einer PETRA-Acquisition von mehreren Minuten ist ein Kontrastmittel bereits homogen im Körper verteilt, bevor die Messung abgeschlossen ist, so dass bei dem Versuch einer Abbildung einer Kontrastmittelinjektion nur unspezifische Mischkontraste erzielbar sind.

Der Wunsch nach immer schnelleren MR-Aufnahmen im klinischen Umfeld führt auf der anderen Seite zu einer Renaissance von Verfahren, bei denen mehrere Bilder simultan aufgenommen werden. Allgemein lassen sich diese Verfahren dadurch charakterisieren, dass zumindest während eines Teils der Messung gezielt Transversalmagnetisierung von zumindest zwei Schichten gleichzeitig für den Bildgebungsprozess genutzt wird ("Multi-Schicht-Bildgebung", "Schicht-Multiplexing", "Simultaneous Multi-Slice" (SMS)). Im Gegensatz dazu wird bei der etablierten "Mehrschicht-Bildgebung" das Signal von zumindest zwei Schichten alternierend, d. h. vollständig unabhängig voneinander mit entsprechender längerer Messzeit aufgenommen.

Bekannte SMS-Verfahren sind beispielsweise Verfahren, die Verfahren aus der parallelen Bildgebung (ppa), bei welchen Wissen über die Sensitivitätsverteilung der bei dem Erfassen der Messdaten eingesetzten Empfangsspulen als zusätzliche Information genutzt wird, um gemäß Nyquist unterabgetastete Messdaten aufzufüllen, in Schicht-Richtung einsetzten, um überlagert aus mehreren Schichten aufgenommene Signale in Signale der einzelnen Schichten zu separieren. Zu diesen Verfahren gehören beispielsweise auch die CAIPIRINHA-Technik, wie sie von Breuer et al. in "Controlled Aliasing in Parallel Imaging Results in Hiher Acceleration (CAIPIRINHA) for Multi-Slice Imaging", Magnetic Resonance in Medicine 53, 2005, S. 684-691 beschrieben ist, und die blipped CAIPIRINHA-Technik, wie sie von Setsompop et al. in "Blipped-Controlled Aliasing in Parallel Imaging for Simultaneous Multislice Echo Planar Imaging With Reduced g-Factor Penalty", Magnetic Resonance in Medicine 67, 2012, S. 1210-1224, beschrieben wird, wobei der im letztgenannten Titel genannte g-Faktor ("g-factor", kurz für "Geometrie-Faktor") ein Maß für eine Trennbarkeit der verschiedenen verwendeten Empfangsspulen darstellt.

Als ein Verfahren diesen g-Faktor weiter zu reduzieren ist es für CAIPIRINHA-Verfahren weiterhin bekannt, die Auslesetrajektorien im k-Raum, und damit das Akquisitionsschema, derart zu verändern, dass die Messdaten entlang von wellenförmig oder schraubenförmig verlaufenden Auslesetrajektorien erfasst werden. Dies ist beispielsweise in dem Artikel von Bilgic et al. "Wave-CAIPI for Highly Accelerated 3D Imaging", Magnetic Resonance in Medicine 73:2152-2162 (2015), oder, für zweidimensionale (2D) Bildgebung in Chen et al. "Self-Calibrating Wave-Encoded Variable-Density Single-Shot Fast Spin Echo Imaging", J. Magn. Reson. Imaging 2018;47:954-966, oder auch für Spinecho(SE)-Verfahren in Gagoski et al. "RARE/Turbo Spin Echo Imaging with Simultaneous Multislice Wave-CAIPI", Magn. Reson. Med. 73:929-938 (2015) beschrieben. Der Erfindung liegt die Aufgabe zugrunde, eine beschleunigte Erfassung von Messdaten mittels Magnetresonanz, die insbesondere besonders kurze Echozeiten TE (z.B. TE < 500 µs) erlaubt, zu ermöglichen, sodass auch Messdaten von Stoffen mit einer transversalen Relaxationszeit T2 in einem Bereich von 30 - 80 µs, wie z.B. Knochen, in einer klinisch akzeptablen Messzeit erfasst werden können.

Die Aufgabe wird gelöst durch ein Verfahren zur Erstellung von Messdaten eines Untersuchungsobjektes mittels Magnetresonanztechnik gemäß Anspruch 1, eine Magnetresonanzanlage gemäß Anspruch 11, ein Computerprogramm gemäß Anspruch 12, sowie einen elektronisch lesbaren Datenträger gemäß Anspruch 13. Ein erfindungsgemäßes Verfahren zur Erstellung von Messdaten eines Untersuchungsobjektes mittels Magnetresonanztechnik umfasst die Schritte:
- Auslesen eines dem Abbildungsgebiet entsprechenden k-Raums, umfassen die Schritte:
   a) Schalten eines konstanten Gradientens (Gᵣ) mittels einer Gradienteneinheit einer Magnetresonanzanlage,
   b) Einstrahlen eines RF-Anregungspulses mittels einer RF-Sende-Empfangsvorrichtung der Magnetresonanzanlage,
   c) nach einer Zeit t1 nach dem zuletzt eingestrahlten RF-Anregungspuls, Aufnehmen von Echosignalen mittels der RF-Sende-Empfangsvorrichtung,
   d) Speichern der aufgenommenen Echosignale als Messdaten entlang der durch die Stärke der durch geschaltete Gradienten vorgegebenen k-Raum-Trajektorie,
      wobei die Schritte a) bis d) wiederholt mit verschiedenen geschalteten konstanten Gradienten (Gₓ) durchgeführt werden, wobei die verschiedenen Gradienten in mindestens zwei Phasenkodierrichtungen geschaltet werden, und wobei mindestens einmal nach der Zeit t1 nach dem zuletzt geschalteten HF-Anregungspuls Zusatzgradienten mit modulierender Amplitude in mindestens einer auf eine durch den bereits geschalteten konstanten Gradienten vorgegebene Richtung senkrecht stehende Richtung geschaltet werden,
      bis der dem Abbildungsgebiet entsprechende k-Raum in einem von der Zeit t1 abhängigen ersten Bereich (B1) ausgelesen ist,
   e) Auslesen des dem Abbildungsgebiet entsprechenden k-Raums, der nicht von dem ersten Bereich (B1) des k-Raums abgedeckt ist (B2), und welcher zumindest das k-Raumzentrum umfasst, auf andere Weise als durch die Schritte a) bis d) beschrieben, und speichern dieser Messdaten,
- Rekonstruieren von Bilddaten aus den aufgenommenen Messdaten des k-Raums mittels einer Steuereinrichtung der Magnetresonanzanlage.

Durch das Schalten von erfindungsgemäßen Zusatzgradienten mit modulierender Amplitude während einer Aufnahme von Messdaten wird die Abdeckung des k-Raums, während der Aufnahme der Messdaten vergrößert. Dadurch werden insgesamt weniger Wiederholungen von Aufnahmen von Messdaten benötigt, um einen gewünschten k-Raum in einer gewünschten Abdeckung abzutasten als ohne die erfindungsgemäßen Zusatzgradienten. Auf diese Weise kann Messzeit eingespart werden.

Eine erfindungsgemäße Magnetresonanzanlage umfasst eine Magneteinheit, eine Gradienteneinheit, eine Hochfrequenzeinheit und eine zur Durchführung eines erfindungsgemäßen Verfahrens ausgebildete Steuereinrichtung mit einer Zusatzgradienteneinheit.

Ein erfindungsgemäßes Computerprogramm implementiert ein erfindungsgemäßes Verfahren auf einer Steuereinrichtung, wenn es auf der Steuereinrichtung ausgeführt wird.

Das Computerprogramm kann hierbei auch in Form eines Computerprogrammprodukts vorliegen, welches direkt in einen Speicher einer Steuereinrichtung ladbar ist, mit Programmcode-Mitteln, um ein erfindungsgemäßes Verfahren auszuführen, wenn das Computerprogrammprodukt in der Recheneinheit des Rechensystems ausgeführt wird.

Ein erfindungsgemäßer elektronisch lesbarer Datenträger umfasst darauf gespeicherte elektronisch lesbare Steuerinformationen, welche zumindest ein erfindungsgemäßes Computerprogramm umfassen und derart ausgestaltet sind, dass sie bei Verwendung des Datenträgers in einer Steuereinrichtung einer Magnetresonanzanlage ein erfindungsgemäßes Verfahren durchführen.

Die in Bezug auf das Verfahren angegebenen Vorteile und Ausführungen gelten analog auch für die Magnetresonanzanlage, das Computerprogrammprodukt und den elektronisch lesbaren Datenträger.

Weitere Vorteile und Einzelheiten der vorliegenden Erfindung ergeben sich aus den im Folgenden beschriebenen Ausführungsbeispielen sowie anhand der Zeichnungen. Die aufgeführten Beispiele stellen keine Beschränkung der Erfindung dar. Es zeigen:
- Fig. 1: ein schematisches Ablaufdiagram eines erfindungsgemäßen Verfahrens,
- Fig. 2: schematisch einen Teil einer Sequenz zur Erfassung von Messdaten,
- Fig. 3: schematisch ein Beispiel eines erfindungsgemäßen Akquisitionsschemas von Messdaten im k-Raum,
- Fig. 4: eine schematisch dargestellte erfindungsgemäße Magnetresonanzanlage.

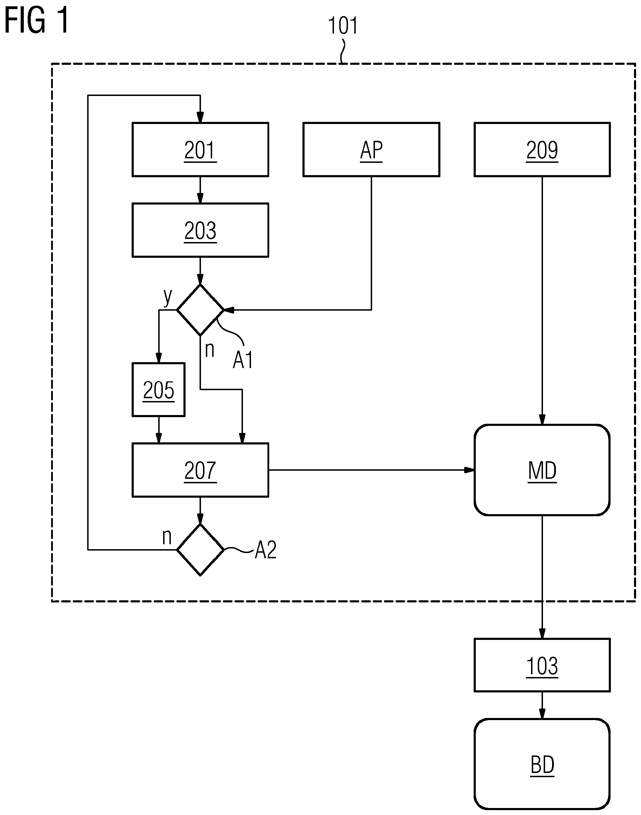

Figur 1 ist ein schematisches Ablaufdiagramm eines erfindungsgemäßen Verfahrens zur Erstellung von Messdaten eines Untersuchungsobjektes mittels Magnetresonanztechnik dargestellt.

Hierbei wird in einem ersten Schritt 101 zur Erstellung eines Bilddatensatzes ein dem Abbildungsgebiet entsprechender k-Raum ausgelesen.

Dazu wird mittels einer Gradienteneinheit einer Magnetresonanzanlage ein konstanter Gradient in eine (für verschiedene Wiederholungen unterschiedliche) Gradientenrichtung Gᵣ geschaltet (Block 201) und ein, insbesondere nicht-schichtselektiver, HF-Anregungspuls mittels einer RF-Sende-Empfangsvorrichtung der Magnetresonanzanlage eingestrahlt (Block 203). Ist der HF-Anregungspuls nicht-schichtselektiv, kann ein dreidimensionales Volumen im k-Raum abgetastet werden, da die Anregung nicht auf eine Schicht beschränkt ist. Die eingestrahlten RF-Anregungspulse werden hierbei z.B. erst nach Erreichen der vollen Stärke des konstanten Gradienten eingestrahlt. Dadurch kann eine durch die Gradienten verursachte Geräuschbildung stark verringert werden.

In einer Abfrage A1 wird entschieden, ob für den zuvor (in Block 201) geschalteten konstanten Gradienten Zusatzgradienten geschaltet werden. Ist dies nicht der Fall (A1, "n") wird mit Block 207 weiterverfahren.

Mindestens einmal sind jedoch nach einem zuvor (in Block 201) geschalteten konstanten Gradienten Zusatzgradienten zu schalten (A1, "y"), sodass für einen solchen zuvor (in Block 201) geschalteten konstanten Gradienten nach der Zeit t1 nach dem zuletzt geschalteten HF-Anregungspuls Zusatzgradienten mit modulierender Amplitude in mindestens einer auf eine durch den bereits geschalteten konstanten Gradienten vorgegebene Richtung senkrecht stehende Richtung geschaltet werden (Block 205) .

Für welche konstanten Gradienten Gᵣ Zusatzgradienten, und wenn ja, welche Zusatzgradienten, zu schalten sind, kann in einer Akquisitionsplanung AP abrufbar festgelegt sein.

Hierbei kann beispielsweise eine maximale Amplitude einer Modulation und/oder ein Verlauf einer Amplitudenentwicklung der Modulation in der dem konstanten Gradienten Gₓ entsprechenden Richtung kₓ im k-Raum von zu schaltenden Zusatzgradienten z.B. in Abhängigkeit einer gewünschten Abdeckung des k-Raums und/oder einer gewählten zulässigen Geräuschentwicklung durch einen Nutzer oder für bestimmte Anwendungen jeweils fest hinterlegt gewählt werden. Beispielsweise ist es denkbar, eine Amplitude mit steigendem Abstand vom k-Raumzentrum ansteigen zu lassen, um eine auf einem Kegelabschnitt des k-Raums verlaufende schraubenförmige k-Raumtrajektorien zu erhalten.

Weiterhin kann ein Anteil des ersten Bereichs vorgegeben werden, in welchem bei einer Aufnahme von Messdaten Zusatzgradienten geschaltet werden. Je größer der Anteil des ersten Bereichs, in welchem bei einer Aufnahme von Messdaten Zusatzgradienten geschaltet werden, desto weniger verschiedene konstante Gradienten und damit desto weniger Wiederholungen der Blöcke 201 bis 207 sind für eine gewünschte Abdeckung des k-Raum in dem ersten Bereich nötig. Die insgesamte Messzeit kann somit entsprechend verkürzt werden. Es kann auch gewählt werden, dass in dem gesamten ersten Bereich, also in einem Anteil von 100% des ersten Bereichs, Zusatzgradienten geschaltet werden. Allerdings wird durch die Zusatzgradienten eine Geräuschbildung durch Wirbelströme wieder erhöht, sodass es sinnvoll sein kann, (ggf. in Abhängigkeit einer maximalen Amplitude der Modulation der Zusatzgradienten) einen maximalen Anteil anzugeben, in welchem bei einer Aufnahme von Messdaten Zusatzgradienten geschaltet werden.

Der Anteil des ersten Bereichs, in welchem bei einer Aufnahme von Messdaten Zusatzgradienten geschaltet werden, kann somit in Abhängigkeit einer zulässigen Geräuschentwicklung und/oder in Abhängigkeit einer gewünschten maximalen Messzeit für die Aufnahme aller Messdaten des ersten Bereichs und/oder in Abhängigkeit einer gewünschten k-Raumabdeckung festgelegt sein.

In jedem Fall werden nach einer Zeit t1 nach dem zuletzt eingestrahlten RF-Anregungspuls werden Echosignale mittels der RF-Sende-Empfangsvorrichtung aufgenommen und als Messdaten MD entlang der durch die Stärke und Richtung der (in Block 201 und ggf. Block 205) geschalteten Gradienten vorgegebenen k-Raumtrajektorie gespeichert (Block 207).

Nachdem alle gewünschten Echosignale nach einem HF-Anregungspuls als Messdaten MD aufgenommen und damit die entsprechenden k-Raum-Trajektorie ausgelesen ist, wird in einer Abfrage A2 geprüft, ob der dem Abbildungsgebiet entsprechende k-Raum in einem von der Zeit t1 abhängigen ersten Bereich bereits ausgelesen ist oder nicht. Wenn nicht ("n"), wird bei Block 201 mit einer erneuten Wiederholung begonnen, wobei bei jeder Wiederholung verschiedene konstante Gradienten in jeweils eine andere Richtung in Block 201 geschaltet werde

Der dem Abbildungsgebiet entsprechende k-Raum, der nicht von dem ersten Bereich des k-Raums abgedeckt ist, welcher erste Bereich mittels der Blöcke 201 bis 207 abgetastet wird, wird zu einem beliebigen Zeitpunkt oder auch zu verschiedenen Zeitpunkten vor, zwischen oder nach dem Auslesen der den ersten Bereich füllenden k-Raum-Trajektorien, z.B. punktweise mittels eines Einzelpunkt-Bildgebungsverfahrens, wie z.B. RASP, oder auf eine andere bekannte Weise, ausgelesen (Block 209) und ebenfalls in als Messdaten MD gespeichert. Werden die Messdaten, welche das k-Raumzentrum enthalten, hierbei kartesisch erfasst, erübrigt sich vor der Rekonstruktion von Bilddaten ein sogenanntes Regridding.

Bei dem Auslesen des dem Abbildungsgebiet entsprechenden k-Raums können die konstanten Gradienten zwischen dem Einstrahlen eines ersten HF-Anregungspulses zur Aufnahme von Messdaten des dem Abbildungsgebiet entsprechenden k-Raums und eines zweiten HF-Anregungspulses zur Aufnahme von weiteren Messdaten des dem Abbildungsgebiet entsprechenden k-Raums kontinuierlich verändert werden. D.h. die konstanten Gradienten werden nicht nach jeder Aufnahme einer k-Raum-Trajektorie heruntergefahren, und für die Aufnahme der nächsten k-Raum-Trajektorie erneut hochgefahren, sondern die konstanten Gradienten werden lediglich von der bereits angenommenen Stärke aus weiter hoch- bzw. heruntergefahren bis die für die nächste Aufnahme erforderliche Stärke erreicht ist. Somit können durch die zur Erzeugung der konstanten Gradienten nötige Bestromung der Gradienteneinheit induzierte Wirbelströme reduziert werden, was die Bildung von Geräuschen reduziert, welche durch die Kräfte verursacht werden, die die Wirbelströme auf die Gradienteneinheit auswirken.

Insbesondere ist es hierbei vorteilhaft, die auszulesenden k-Raum-Trajektorien derart anzuordnen, dass die Stärke der konstanten Gradienten jeweils nur möglichst gering verändert werden muss, wodurch die durch die Änderung der verschiedenen konstanten Gradienten verursachten Geräusche weiter reduziert werden können.

In einem weiteren Schritt 102 kann aus den aufgenommenen Echosignalen welche als Messdaten MD gespeichert sind, z.B. mittels der Steuereinrichtung der Magnetresonanzanlage, unter Verwendung einer Fourier-Transformation, und ggf. unter Verwendung von Regridding-Verfahren, ein Bilddatensatz BD rekonstruiert werden, der ebenfalls wie die Messdaten MD gespeichert und/oder angezeigt werden kann.

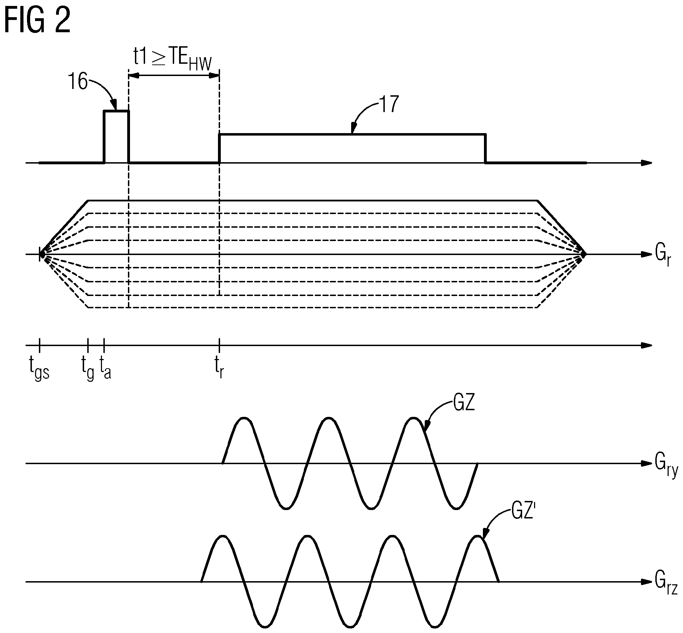

In Figur 2 ist schematisch der Teil einer erfindungsgemäßen Sequenz, der zur Erfassung von Messdaten in einem ersten Bereich dienen kann, dargestellt (vgl. Figur 1, Blöcke 201 bis 205 bzw. 207). Zu einem Zeitpunkt t_{gs} wird ein Gradient zur Phasenkodierung in Gradientenrichtung Gᵣ hochgefahren, wobei sich die Gradientenrichtung aus den Richtungen (z.B. mindestens zwei Phasenkodierrichtungen Gx, Gy, Gz) ergibt, in welche der Gradient geschaltet wird, und erreicht zu einem Zeitpunkt t_{g} seine volle Stärke, die konstant gehalten wird. Zu einem späteren Zeitpunkt tₐ > t_{g} wird ein HF-Anregungspuls 16 eingestrahlt. Nach einer Echozeit t1 nach dem HF-Anregungspuls 16, die, z.B. für ultrakurze Echozeiten, der hardwaregegebenen minimalen Umschaltzeit zwischen einem Sende-Modus und einem Empfangs-Modus einer verwendeten HF-Sende-Empfangsvorrichtung TE_{HW} entspricht, wird zum Zeitpunkt tᵣ eine Auslesezeitspanne 17 zum Auslesen der Echosignale und damit zur Aufnahme der Messdaten begonnen.

In dem in Figur 2 dargestellten Ausführungsbeispiel wird der konstante Gradient in Phasenkodierrichtung geschaltet bevor der HF-Anregungspuls 16 eingestrahlt wird.

Zusatzgradienten mit modulierender Amplitude GZ, GZ' können z.B. in mindestens eine auf die Gradientenrichtung Gr senkrecht stehende Richtung G_{ry} und/oder G_{rz} geschaltet werden, wobei auch die Richtungen G_{ry} und G_{rz} jeweils senkrecht aufeinander stehen. Zusatzgradienten GZ, GZ' werden derart geschaltet, dass sie in der Auslesezeitspanne 17, während der Aufnahme der Messdaten, wirken.

Wird nur ein Zusatzgradient GZ oder GZ' oder zwei Zusatzgradienten GZ und GZ' mit gleicher Phasenlage und gleicher Modulation (nicht dargestellt) geschaltet, wird die während der Aufnahme der Messdaten durchlaufene k-Raumtrajektorie der Modulation entsprechend, z.B. wellenförmig, in einer durch die Gradientenrichtung Gᵣ und die Richtungen G_{ry} und/oder G_{rz}, in die Zusatzgradienten GZ, GZ' geschaltet sind, festgelegten Ebene von der Gradientenrichtung Gᵣ abgelenkt. Die Modulation der Amplitude eines Zusatzgradienten GZ, GZ' kann beispielsweise, wie dargestellt, sinusförmig sein. Eine sinusförmige Modulation führt zu einer gleichförmigen, periodischen Variation der Amplitude, die sich gut berechnen und erzeugen lässt.

Werden zwei Zusatzgradienten GZ und GZ' mit unterschiedlicher Phasenlage oder unterschiedlicher Modulation geschaltet, wird die während der Aufnahme der Messdaten durchlaufene k-Raumtrajektorie der bzw. den Modulationen entsprechend, z.B. schraubenförmig (ggf. elliptisch) um die Gradientenrichtung Gᵣ herum verlaufen.

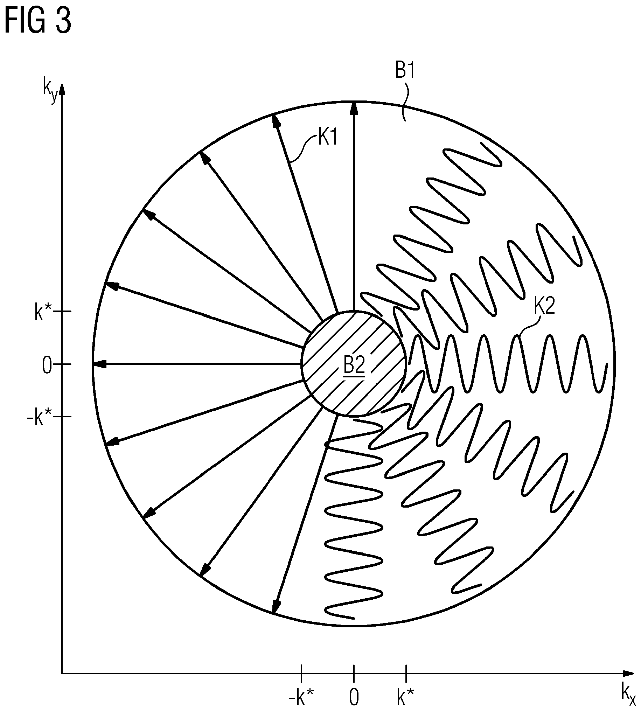

Figur 3 zeigt schematisch ein Beispiel eines erfindungsgemäßen Akquisitionsschemas von Messdaten im k-Raum. Die, z.B. mittels einer Sequenz gemäß Figur 2 auslesbaren k-Raum-Werte in dem ersten Bereich B1, starten erst bei einem von der Stärke der anliegenden Gradienten und insbesondere von der Echozeit t1 abhängigen k-Raum-Wert k*. Es gilt: *k*^{∗}=*t*1^{∗}*G,* mit G die Stärke (Amplitude) des Gradienten.

Bei dem in Figur 3 dargestellten Abtastschema kann es sich um ein zweidimensionales Abtastschema, z.B. in ky-kx-Ebene (wie dargestellt), oder auch um ein dreidimensionales Abtastschema, das sich analog kugelförmig um das k-Raumzentrum erstreckt, handeln. In Figur 3 sind in einem ersten Bereich B1, der sich im k-Raum zwischen einem Mindestradius von k* bis zu einem Radius eines maximalen k-Raumwerts (äußerer Rand B1) um das k-Raumzentrum herum erstreckt, k-Raumtrajektorien einer ersten Art K1 und k-Raumtrajektorien einer zweiten Art K2 dargestellt.

Die k-Raumtrajektorien der ersten Art K1 verlaufen auf radialen Halbspeichen von dem in Richtung der jeweiligen geschalteten konstanten Gradienten Gᵣ k* vom k-Raumzentrum entfernten k-Raumpunkt radial nach außen. Bei einer Aufnahme von Messdaten entlang einer der k-Raumtrajektorien der ersten Art K1 wurden keine Zusatzgradienten geschaltet. k-Raumtrajektorien der zweiten Art K2 verlaufen ebenfalls von einem k* von dem k-Raumzentrum entfernten k-Raumpunkt bis zum äußeren Rand des ersten Bereichs B1, jedoch nicht auf einer radialen k-Raumtrajektorie, sondern auf einer gemäß den geschalteten Zusatzgradienten modulierten k-Raumtrajektorie. Zu dem Zusammenhang zwischen geschalteten Zusatzgradienten und Verlauf der k-Raumtrajektorie, wird auf die mit Bezug auf Figur 2 bereits gemachten Ausführungen verwiesen.

Es ist ersichtlich, dass der k-Raum mit einer k-Raumtrajektorie der zweiten Art K2 mit einer höheren Abdeckung abgetastet wird, als mit einer k-Raumtrajektorie der ersten Art K1. In dem in Figur 3 dargestellten Beispiel wird etwa die Hälfte des ersten Bereichs B1 mit k-Raumtrajektorien der ersten Art K1 und die andere Hälfte mit k-Raumtrajektorien der zweiten Art K2 abgedeckt. Durch die in Abhängigkeit der maximalen Amplituden der geschalteten Zusatzgradienten ausgelenkte Form der k-Raumtrajektorien zweiter Art K2 ist eine geringere Anzahl an k-Raumtrajektorien der zweiten Art K2 nötig, um eine gleich Abdeckung des k-Raums zu erreichen, als k-Raumtrajektorien der ersten Art K1 nötig wären. Es ist denkbar, bei jeder Aufnahme von Messdaten im ersten Bereich B1 Zusatzgradienten zu schalten, sodass im ersten Bereich B1 nur k-Raumtrajektorien der zweiten Art K2 abgetastet werden. Es ist auch denkbar, dass für zumindest eine k-Raumtrajektorie der zweiten Art K2, entlang derer Messdaten aufgenommen werden, auch entlang einer k-Raumtrajektorie der ersten Art K1 mit demselben konstanten Gradienten Gₓ Messdaten aufgenommen werden, sodass die Messdaten entlang der k-Raumtrajektorie der ersten Art K1 im k-Raum im Inneren der k-Raumtrajektorie der zweiten Art K2 liegen. Es kann auch der gesamte erste Bereich B1 durch derartige Paare von k-Raumtrajektorien der ersten und der zweiten Art K1 und K2 mit jeweils gleichen konstanten Gradienten Gₓ abgetastet werden.

k-Raum-Punkte im k-Raumzentrum könnten mit einer Sequenz, wie sie in Figur 2 dargestellt ist, nur erfasst werden, wenn die Zeit t1 gegen Null ginge, da der konstante Gradient bereits vor dem Einstrahlen des HF-Anregungspulses seine volle Stärke erreicht hat. Dies ist in realen Systemen jedoch in aller Regel nicht möglich, sodass Messdaten in dem das k-Raumzentrum umfassenden zweiten Bereich B2, der dem Bereich entspricht, der den dem Abbildungsgebiet entsprechenden k-Raum abdeckt, der nicht von dem ersten Bereich B1 des k-Raums abgedeckt ist, auf andere Weise als es im ersten Bereich B1 der Fall ist. Beispielsweise können Messpunkte in dem zweiten Bereich B2 wie in Verbindung mit Figur 1, Block 209 beschrieben z.B. punktweise mittels Einzelpunkt-Bildgebungsverfahren auf einem kartesischen k-Raum-Gitter oder auf andere bekannte Weise erfasst werden. Grundsätzlich ist auch für Messdaten, die im k-Raum in dem zweiten Bereich B2 liegen denkbar, diese auf radialen k-Raumtrajektorien aufzunehmen.

In dem in Figur 3 dargestellten Beispiel liegen die k-Raumtrajektorien zweiter Art in einem zusammenhängenden Teilbereich des ersten Bereichs B1. Dies kann erreicht werden, indem Zusatzgradienten während Aufnahmen von Messdaten in einem zusammenhängenden Teilbereich des ersten Bereichs entsprechenden Gradientenrichtungen Gᵣ geschaltet werden. Auf diese Weise kann die Rekonstruktion von Bilddaten aus den im k-Raum aufgenommenen Messdaten ebenfalls zumindest in Unterschritten bereichsweise erfolgen.

Es ist jedoch auch denkbar, k-Raumtrajektorien der ersten Art K1 und k-Raumtrajektorien der zweiten Art K2 gleichverteilt im ersten Bereich anzuordnen, indem Zusatzgradienten während Aufnahmen von Messdaten in gleichverteilte Gradientenrichtungen Gᵣ geschaltet werden.

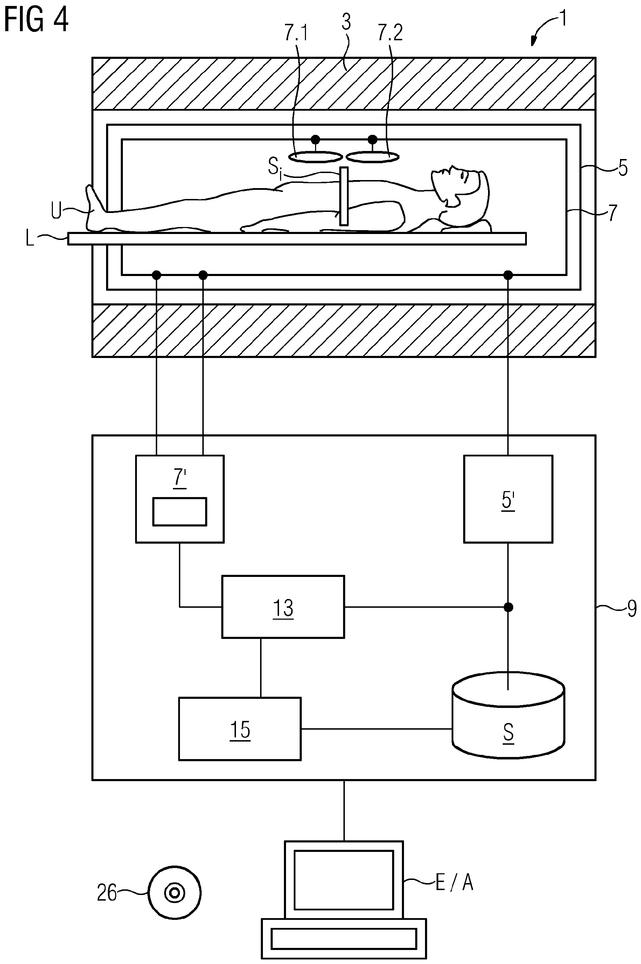

Figur 4 stellt schematisch eine erfindungsgemäße Magnetresonanzanlage 1 dar. Diese umfasst eine Magneteinheit 3 zur Erzeugung des Grundmagnetfeldes, eine Gradienteneinheit 5 zur Erzeugung der Gradientenfelder, eine Hochfrequenzeinheit 7 zur Einstrahlung und zum Empfang von Hochfrequenzsignalen und eine zur Durchführung eines erfindungsgemäßen Verfahrens ausgebildete Steuereinrichtung 9.

In der Figur 4 sind diese Teileinheiten der Magnetresonanzanlage 1 nur grob schematisch dargestellt. Insbesondere kann die Hochfrequenzeinheit 7 aus mehreren Untereinheiten, beispielsweise aus mehreren Spulen wie den schematisch gezeigten Spulen 7.1 und 7.2 oder mehr Spulen bestehen, die entweder nur zum Senden von Hochfrequenzsignalen oder nur zum Empfangen der ausgelösten Hochfrequenzsignale oder für beides ausgestaltet sein können.

Zur Untersuchung eines Untersuchungsobjektes U, beispielsweise eines Patienten oder auch eines Phantoms, kann dieses auf einer Liege L in die Magnetresonanzanlage 1 in deren Messvolumen eingebracht werden. Die Schicht Sᵢ stellt ein exemplarisches Zielvolumen des Untersuchungsobjekts dar, aus dem Daten aufgenommen und damit erfasst werden sollen.

Die Steuereinrichtung 9 dient der Steuerung der Magnetresonanzanlage 1 und kann insbesondere die Gradienteneinheit 5 mittels einer Gradientensteuerung 5' und die Hochfrequenzeinheit 7 mittels einer Hochfrequenz-Sende-/Empfangs-Steuerung 7' steuern. Die Hochfrequenzeinheit 7 kann hierbei mehrere Kanäle umfassen, auf denen Signale gesendet oder empfangen werden können.

Die Hochfrequenzeinheit 7 ist zusammen mit ihrer Hochfrequenz-Sende-/Empfangs-Steuerung 7' für die Erzeugung und das Einstrahlen (Senden) eines Hochfrequenz-Wechselfeldes zur Manipulation der Spins in einem zu manipulierenden Bereich (beispielsweise in zu messenden Schichten Sᵢ) des Untersuchungsobjekts U zuständig. Dabei wird die Mittenfrequenz des, auch als B1-Feld bezeichneten, Hochfrequenz-Wechselfeldes in aller Regel möglichst so eingestellt, dass sie nahe der Resonanzfrequenz der zu manipulierenden Spins liegt. Abweichungen von der Mittenfrequenz von der Resonanzfrequenz werden als Off-Resonanz bezeichnet. Zur Erzeugung des B1-Feldes werden in der Hochfrequenzeinheit 7 mittels der Hochfrequenz-Sende-/Empfangs-Steuerung 7' gesteuerte Ströme an den HF-Spulen angelegt.

Weiterhin umfasst die Steuereinrichtung 9 eine Zusatzgradienteneinheit 15, mit welcher erfindungsgemäße Zusatzgradienten bestimmt werden können, die durch die Gradientensteuerung 5' umgesetzt werden können. Die Steuereinrichtung 9 ist insgesamt dazu ausgebildet, ein erfindungsgemäßes Verfahren durchzuführen.

Eine von der Steuereinrichtung 9 umfasste Recheneinheit 13 ist dazu ausgebildet alle für die nötigen Messungen und Bestimmungen nötigen Rechenoperationen auszuführen. Hierzu benötigte oder hierbei ermittelte Zwischenergebnisse und Ergebnisse können in einer Speichereinheit S der Steuereinrichtung 9 gespeichert werden. Die dargestellten Einheiten sind hierbei nicht unbedingt als physikalisch getrennte Einheiten zu verstehen, sondern stellen lediglich eine Untergliederung in Sinneinheiten dar, die aber auch z.B. in weniger oder auch in nur einer einzigen physikalischen Einheit realisiert sein können.

Über eine Ein-/Ausgabeeinrichtung E/A der Magnetresonanzanlage 1 können, z.B. durch einen Nutzer, Steuerbefehle an die Magnetresonanzanlage geleitet werden und/oder Ergebnisse der Steuereinrichtung 9 wie z.B. Bilddaten angezeigt werden.

Ein hierin beschriebenes Verfahren kann auch in Form eines Computerprogrammprodukts vorliegen, welches ein Programm umfasst und das beschriebene Verfahren auf einer Steuereinrichtung 9 implementiert, wenn es auf der Steuereinrichtung 9 ausgeführt wird. Ebenso kann ein elektronisch lesbarer Datenträger 26 mit darauf gespeicherten elektronisch lesbaren Steuerinformationen vorliegen, welche zumindest ein solches eben beschriebenes Computerprogrammprodukt umfassen und derart ausgestaltet sind, dass sie bei Verwendung des Datenträgers 26 in einer Steuereinrichtung 9 einer Magnetresonanzanlage 1 das beschriebene Verfahren durchführen.

## Patentansprüche

1. Verfahren zur Erstellung von Messdaten eines Untersuchungsobjektes mittels Magnetresonanztechnik, umfassend die Schritte:
- Auslesen eines dem Abbildungsgebiet entsprechenden k-Raums, umfassend die Schritte:
a) Schalten eines konstanten Gradientens (Gᵣ) mittels einer Gradienteneinheit einer Magnetresonanzanlage,
b) Einstrahlen eines RF-Anregungspulses mittels einer RF-Sende-Empfangsvorrichtung der Magnetresonanzanlage,
c) nach einer Zeit t1 nach dem zuletzt eingestrahlten RF-Anregungspuls, Aufnehmen von Echosignalen mittels der RF-Sende-Empfangsvorrichtung,
d) Speichern der aufgenommenen Echosignale als Messdaten entlang der durch die Stärke der durch geschaltete Gradienten vorgegebenen k-Raum-Trajektorie,
wobei die Schritte a) bis d) wiederholt mit verschiedenen geschalteten konstanten Gradienten (Gᵣ) durchgeführt werden, wobei die verschiedenen Gradienten in mindestens zwei Phasenkodierrichtungen geschaltet werden, und wobei mindestens einmal nach der Zeit t1 nach dem zuletzt geschalteten HF-Anregungspuls Zusatzgradienten mit modulierender Amplitude in mindestens einer auf eine durch den bereits geschalteten konstanten Gradienten vorgegebene Richtung senkrecht stehende Richtung geschaltet werden,
bis der dem Abbildungsgebiet entsprechende k-Raum in einem von der Zeit t1 abhängigen ersten Bereich (B1) ausgelesen ist,
e) Auslesen des dem Abbildungsgebiet entsprechenden k-Raums, der nicht von dem ersten Bereich (B1) des k-Raums abgedeckt ist (B2), und welcher zumindest das k-Raumzentrum umfasst, auf andere Weise als durch die Schritte a) bis d) beschrieben, und speichern dieser Messdaten,
- Rekonstruieren von Bilddaten aus den aufgenommenen Messdaten des k-Raums mittels einer Steuereinrichtung der Magnetresonanzanlage.

2. Verfahren nach Anspruch 1, wobei die eingestrahlten RF-Anregungspulse nach Erreichen der vollen Stärke des konstanten Gradienten eingestrahlt werden.

3. Verfahren nach einem der vorhergehenden Ansprüche, wobei die eingestrahlten RF-Anregungspulse nicht-schichtselektive RF-Anregungspulse sind.

4. Verfahren nach einem der vorhergehenden Ansprüche, wobei die Messdaten im Schritt e) als kartesische Messdaten, insbesondere mittels eines Einzelpunkt-Bildgebungsverfahrens, ausgelesen werden.

5. Verfahren nach einem der vorhergehenden Ansprüche, wobei eine Modulation der Amplitude eines Zusatzgradienten sinusförmig ist.

6. Verfahren nach einem der vorhergehenden Ansprüche, wobei eine maximale Amplitude der Modulation der Zusatzgradienten in Abhängigkeit einer gewünschten Abdeckung des k-Raums gewählt ist.

7. Verfahren nach einem der vorhergehenden Ansprüche, wobei ein Anteil des ersten Bereichs vorgegeben wird, in welchem bei einer Aufnahme von Messdaten Zusatzgradienten geschaltet werden.

8. Verfahren nach Anspruch 7, wobei der Anteil des ersten Bereichs, in welchem bei einer Aufnahme von Messdaten Zusatzgradienten geschaltet werden in Abhängigkeit einer zulässigen Geräuschentwicklung und/oder in Abhängigkeit einer gewünschten maximalen Messzeit für die Aufnahme aller Messdaten des ersten Bereichs und/oder in Abhängigkeit einer gewünschten k-Raumabdeckung festgelegt ist.

9. Verfahren nach einem der vorhergehenden Ansprüche, wobei Zusatzgradienten während Aufnahmen von Messdaten in einem zusammenhängenden Teilbereich des ersten Bereichs oder gleichverteilt im ersten Bereich geschaltet werden.

10. Verfahren nach einem der vorhergehenden Ansprüche, wobei während jeder Aufnahme von Messdaten Zusatzgradienten geschaltet werden.

11. Magnetresonanzanlage (1) umfassend, eine Magneteinheit (3), eine Gradienteneinheit (5), eine Hochfrequenzeinheit (7) und eine Steuereinrichtung (9) mit einer Hochfrequenz-Sende-/Empfangs-Steuerung (7') mit einer Zusatzgradienteneinheit (15), wobei die Steuereinrichtung (9) dazu ausgebildet ist, ein Verfahren nach einem der Ansprüche 1 bis 10 auf der Magnetresonanzanlage (1) auszuführen.

12. Computerprogramm, welches direkt in einen Speicher einer Steuereinrichtung (9) einer Magnetresonanzanlage (1) ladbar ist, mit Programm-Mitteln, um die Schritte des Verfahrens nach einem der Ansprüche 1 bis 10 auszuführen, wenn das Programm in der Steuereinrichtung (9) der Magnetresonanzanlage (1) ausgeführt wird.

13. Elektronisch lesbarer Datenträger mit darauf gespeicherten elektronisch lesbaren Steuerinformationen, welche zumindest ein Computerprogramm nach Anspruch 12 umfassen und derart ausgestaltet sind, dass sie bei Verwendung des Datenträgers in einer Steuereinrichtung (9) einer Magnetresonanzanlage (1) ein Verfahren nach einem der Ansprüche 1 bis 10 durchführen.

## Claims

1. Method for producing scan data from an examination object by means of magnetic resonance technology, comprising the steps:
- reading out a k-space which corresponds to the mapping area, comprising the steps:
a) switching a constant gradient (Gᵣ) by means of a gradient unit of a magnetic resonance system,
b) irradiating an RF excitation pulse by means of an RF transmit-receive device of the magnetic resonance system,
c) following a time t1 after the last irradiated RF excitation pulse, recording echo signals by means of the RF transmit-receive device,
d) storing the recorded echo signals as scan data along the k-space trajectory predetermined by the strength of the through-connected gradients,
wherein the steps a) to d) are carried out repeatedly with various switched constant gradients (Gᵣ), wherein the various gradients are switched in at least two phase encoding directions, and wherein at least once following the time t1 after the last switched HF excitation pulse, additional gradients with a modulating amplitude are switched in at least one direction which is oriented at right angles to a direction predetermined by the already switched constant gradients,
until the k-space corresponding to the imaging area is read out in a first region (B1) which depends on the time t1,
e) reading out the k-space corresponding to the mapping area, which is not covered (B2) by the first region (B1) of the k-space and which comprises at least the k-space centre, differently to that described by steps a) to d), and storing this scan data,
- reconstructing image data from the recorded scan data of the k-space by means of a control facility of the magnetic resonance system.

2. Method according to claim 1, wherein the irradiated RF excitation pulses are irradiated after the full strength of the constant gradients is reached.

3. Method according to one of the preceding claims, wherein the irradiated RF excitation pulses are non-slice-selective RF excitation pulses.

4. Method according to one of the preceding claims, wherein the scan data is read out in step e) as Cartesian scan data, in particular by means of a single point imaging method.

5. Method according to one of the preceding claims, wherein a modulation of the amplitude of an additional gradient is sinusoidal.

6. Method according to one of the preceding claims, wherein a maximum amplitude of the modulation of the additional gradients is selected as a function of a desired coverage of the k-space.

7. Method according to one of the preceding claims, wherein a portion of the first region is predetermined, in which additional gradients are switched while scan data is recorded.

8. Method according to claim 7, wherein the portion of the first region, in which additional gradients are switched while scan data is recorded, is defined as a function of a permissible noise development and/or as a function of a desired maximum scan time for the recording of all scan data of the first region and/or as a function of a desired k-space coverage.

9. Method according to one of the preceding claims, wherein additional gradients are switched during recordings of scan data in an associated subregion of the first region or equally distributed in the first region.

10. Method according to one of the preceding claims, wherein additional gradients are switched during each recording of scan data.

11. Magnetic resonance system (1) comprising a magnet unit (3), a gradient unit (5), a high frequency unit (7) and a control facility (9) with a high frequency transmit/receive controller (7') with an additional gradient unit (15), wherein the control facility (9) is embodied to carry out a method according to one of claims 1 to 10 on the magnetic resonance system (1).

12. Computer program, which can be loaded directly into a memory store of a control facility (9) of a magnetic resonance system (1), having program means, in order to carry out the steps of the method according to one of claims 1 to 10, if the program is executed in the control facility (9) of the magnetic resonance system (1).

13. Electronically readable data storage medium with electronically readable control information stored thereupon, which comprises at least one computer program according to claim 12, and is configured so that it carries out a method according to one of claims 1 to 10 when the data storage medium in a control facility (9) of a magnetic resonance system (1) is used.

## Revendications

1. Procédé d'établissement de données de mesure d'un objet à examiner au moyen de la technique de la résonnance magnétique, comprenant les stades :
- lecture d'un espace k correspondant au domaine de reproduction, comprenant les stades :
a) mise d'un gradient (Gᵣ) constant au moyen d'une unité de gradient d'une installation de résonnance magnétique,
b) exposition à une impulsion d'excitation RF au moyen d'un système d'émission - réception RF de l'installation de résonnance magnétique,
c) après un temps t1 après l'impulsion d'excitation RF d'exposition en dernier, enregistrement de signaux d'écho au moyen du système d'émission - réception RF,
d) mise en mémoire des signaux d'écho enregistrés comme données de mesure le long de la trajectoire d'espace k donnée à l'avance par l'intensité des gradients mis,
dans lequel on effectue les stades a) à d) de manière répétée en mettant des gradients (Gᵣ) constants différents, dans lequel on met les gradients différents dans au moins deux directions de codage de phase et dans lequel, au moins une fois après le temps t1, après l'impulsion d'excitation HF mise en dernier, on met des gradients supplémentaires d'amplitude modulante dans au moins une direction perpendiculaire à une direction donnée à l'avance par les gradients constants déjà mis, jusqu'à ce que l'espace k correspondant au domaine de reproduction soit lu dans une première partie (B1), qui dépend du temps t1,
e) lecture de l'espace k correspondant au domaine de reproduction, qui n'est pas recouvert (B2) par la première partie (B1) de l'espace k et qui comprend au moins le centre de l'espace k, d'une autre façon que décrite par les stades a) à d) et mis en mémoire de ces données de mesure,
- reconstruction de données d'image à partir des données de mesure enregistrées de l'espace k au moyen d'un dispositif de commande de l'installation de résonnance magnétique.

2. Procédé suivant la revendication 1, dans lequel on expose aux impulsions d'excitation RF d'exposition après avoir atteint la pleine intensité du gradient constant.

3. Procédé suivant l'une des revendications précédentes, dans lequel les impulsions d'excitation RF d'exposition sont des impulsions d'excitation RF non sélectives par tranche.

4. Procédé suivant l'une des revendications précédentes, dans lequel on lit, notamment au moyen d'un procédé d'imagerie à points individuels, les données de mesure au stade e) sous la forme de données de mesure cartésiennes.

5. Procédé suivant l'une des revendications précédentes, dans lequel une modulation de l'amplitude d'un gradient supplémentaire est sinusoïdale.

6. Procédé suivant l'une des revendications précédentes, dans lequel une amplitude maximum de la modulation des gradients supplémentaires est sélectionnée en fonction d'une couverture souhaitée de l'espace k.

7. Procédé suivant l'une des revendications précédentes, dans lequel on prescrit une proportion de la première partie, dans laquelle on met des gradients supplémentaires, lors d'un enregistrement de données de mesure.

8. Procédé suivant la revendication 7, dans lequel, la proportion de la première partie, dans laquelle, lors de l'enregistrement de données de mesure, on met des gradients supplémentaires, est fixée en fonction d'une production de bruits admissible et/ou en fonction d'un temps de mesure maximum souhaité pour l'enregistrement de toutes les données de mesure de la première partie et/ou en fonction d'une couverture souhaitée de l'espace k.

9. Procédé suivant l'une des revendications précédentes, dans lequel on met, dans une partie partielle d'un seul tenant de la première partie ou d'une manière répartie uniformément dans la première partie, des gradients supplémentaires pendant l'enregistrement de données de mesure.

10. Procédé suivant l'une des revendications précédentes, dans lequel on met des gradients supplémentaires pendant chaque enregistrement de données de mesure.

11. Installation (1) de résonnance magnétique comprenant une unité (3) d'aimant, une unité (5) de gradient, une unité (7) de haute fréquence et un dispositif (9) de commande ayant une commande (7') d'émission / réception de haute fréquence, comprenant une unité (15) de gradient supplémentaire, dans lequel le dispositif (9) de commande est constitué pour effectuer un procédé suivant l'une des revendications 1 à 10 sur l'installation (1) de résonnance magnétique.

12. Programme d'ordinateur, qui peut être chargé directement dans la mémoire d'un dispositif (9) de commande d'une installation (1) de résonnance magnétique, comprenant des moyens de programme pour effectuer les stades du procédé suivant l'une des revendications 1 à 10, lorsque le programme est exécuté dans le dispositif (9) de commande de l'installation (1) de résonnance magnétique.

13. Support de données, déchiffrable électroniquement, comprenant des informations de commande déchiffrables électroniquement, qui sont mises en mémoire et qui comprennent au moins un programme d'ordinateur suivant la revendication 12 et qui sont conformées de manière à effectuer un procédé suivant l'une des revendications 1 à 10 lors d'une utilisation du support de données dans un dispositif (9) de commande d'une installation (1) de résonnance magnétique.
